# EUROPEAN PATENT SPECIFICATION

(11) **EP 4 406 944 B1**
(45) Date of publication and mention of the grant of the patent: **29.10.2025**
(21) Application number: 23153617.8
(22) Date of filing: 27.01.2023
(51) Int. Cl.: C07D 307/77, C07C 213/02, C07C 217/84, C07C 217/92, C07C 217/94, C07D 307/91, H10K 30/00, H10K 85/60

(54) **SPIRO-TYPE HOLE TRANSPORT MATERIALS WITH EXTENDED EDGE PI-CONJUGATION LENGTH**
SPIRO-LOCHTRANSPORTMATERIALIEN MIT VERLÄNGERTER PI-KONJUGATIONSLÄNGE AM RAND
MATÉRIAUX DE TRANSPORT DE TROUS DE TYPE SPIRO AYANT UNE LONGUEUR DE CONJUGAISON PI À BORD ÉTENDU

(43) Date of publication of application: 31.07.2024
(73) Proprietor: TOYOTA JIDOSHA KABUSHIKI KAISHA, Aichi-Ken 471-8571 (JP)
(72) Inventor: KINGE, Sachin, 1140 Brussels (BE); DING, Yong, 1020 Renens (CH); LIU, Xuepeng, 1020 Renens (CH); DING, Bin, 1950 Sion (CH); NAZEERUDDIN, Mohammad Khaja, 1024 Ecublens (CH); DYSON, Paul Joseph, 1024 Ecublens (CH)
(74) Representative: Cabinet Beau de Loménie

(56) References cited:
- EP-A1- 2 180 029
- WO-A1-2021/167214
- WO-A1-2022/249789
- CN-A- 103 589 419
- CN-A- 105 859 678
- CN-A- 109 879 767
- CN-A- 111 689 867
- CN-A- 115 215 754
- CN-A- 115 490 632
- JP-A- 2011 113 650
- JP-A- 2012 023 266
- US-A1- 2018 197 688
- US-A1- 2019 288 207

## Description

### BACKGROUND OF THE INVENTION

### 1. Field of the Invention

The present invention relates to organic hole transporting materials (HTMs) useful to increase power conversion efficiency (PCE) of perovskite solar cells (PSCs).

### 2. Description of Related Art

The power conversion efficiency (PCE) of perovskite solar cells (PSCs) has recently increased from the initial 3.8% to a certified 25.7%. These achievements are attributed to the unique photoelectric characteristics of organic-inorganic perovskites, such as appropriate direct band gap to make full use of solar energy and high charge mobility/long carrier diffusion length for effective photogenerated carrier extraction and transport. Generally, most high-performance PSCs can also be simplified as having three key parts: 1) the metal oxide-based electron-transporting material, 2) the organic hole transporting material (HTM), and 3) the perovskite absorber group.

Among the critical materials of PSCs, the HTM has an important effect on efficiency and stability. However, there are only limited options for HTM materials with good properties. The conventional HTM material used is spiro-OMeTAD, which has the following structure:

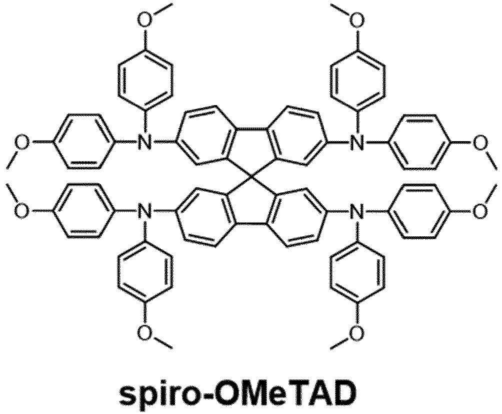

Spiro-OMeTAD maintains the highest device PCE due to the advantages of the unique twisted spiro core, which may grant suitable energy levels for transferring holes and blocking electrons. The molecular structures of HTMs have a significant effect on PSC performance. Therefore, it would be of interest to obtain efficient and stable PSCs through molecular engineering of spiro-typed HTMs. Generally, the ideal HTMs for efficient and stable PSCs should meet suitable energy levels, and show high hole mobility and thermal stability. A closer HOMO energy level between HTMs and perovskite would result in minor voltage loss of PSCs. A higher hole mobility would accelerate the hole transfer to the electrode. A higher glass transition temperature (T_{g}) would enhance the thermal stability of PSCs. Ref: 10.1039/d1cs01157j; 10.1039/C8TA08503J; https://www.nature.com/articles/s41560-018-0200-6. Previously, the replacement of some functional groups of the partial anisole on spiro-OMeTAD has been proven as the way to enhance PSCs' performance, such as meta-anisole, [10.1021/ja502824c] methoxynaphthalene, [https://doi.org/10.1038/s41566-021-00931-7] fluorine functionalized anisole [Science, 2020, 369, 1615.], Ref: 10.1039/d2tc02564g. Although the developed new HTMs show a higher PCE than spiro-OMeTAD in operation, the highest PCE is lower than 25%. Moreover, it is noteworthy that spiro-OMeTAD still retains the highest reported PCE (25.8%) in PSCs [https://doi.org/10.1038/s41586-021-03964-8]. Therefore, it is promising and necessary to further design and develop novel efficient spiro-type HTMs with suitable energy level, high hole mobility and high T_{g} to enhance the performance of PSCs.

HTMs with extended π-conjugation have been reported [J. Mater. Chem. C, 2018, 6, 8280; Chem. Sci., 2014, 5, 2702; Chem. Sci., 2016, 7, 6068; Dyes Pigments, 2017, 139, 129; https://doi.org/10.1038/s41560-018-0200-6]. In the present invention, it has been intended to design and develop a series of facile spiro-typed HTM by extending the edge π-conjugation length. In some developed materials, extending the edge π-conjugation of spiro-OMeTAD could decrease HOMO level, enhance the hole mobility, conductivity as well as T_{g} value.

CN 115 215 754 discloses compounds having the following general formula:

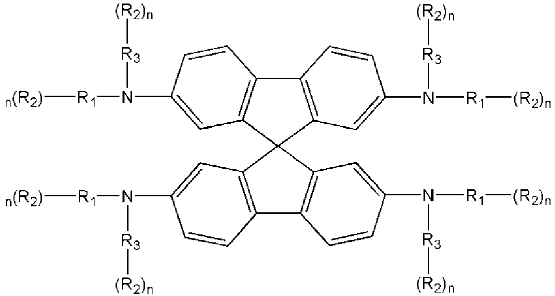

CN 115215 754 discloses in particular compounds with the spiro core structure above formula, with the N atoms bearing phenyl, naphthyl or biphenyl rings that may have -OMe substituents.

WO 2021/167214 and CN 111 689 867 disclose compounds with the same spiro core structure as in the above formula, with the N atoms bearing phenyl or naphthyl rings that may have -OMe substituents. JP 2012 023266 disclose compounds with the same spiro core structure as in the above formula, with the N atoms bearing phenyl or biphenyl rings that may have -OMe substituents.

### SUMMARY OF THE INVENTION

The invention described herein provides spiro-type HTM compounds with an extension of the edge π-conjugation length, as well as methods of synthesis of these compounds and perovskite solar cells containing the spiro-typed HTM compounds.

The spiro-type HTM compounds of the present invention contain the spirobifluorene helical structure as the core, and extend the terminal structure of the spirobifluorene molecule to improve the conjugation of the spirobifluorene molecule. It is postulated that this will lower the HOMO energy level, thereby reducing the open-circuit voltage (*V*_{oc}) loss of PSCs, enhancing the hole extraction ability, and improving the photoelectric conversion efficiency (PCE). Moreover, the extended molecular terminal structure increases the volume of the compound molecule, so that the glass transition temperature of the compound may be enhanced, and may thereby improve the stability of the HTM compounds of the present invention. Therefore, the spiro-type HTMs with an extended edge π-conjugation length, having a low HOMO energy level, may show excellent electrical conductivity and excellent thermal stability, and when these spiro-type HTMs are used in a PSC hole transport layer, they may increase the photovoltaic performance and stability of PSC devices. Such PSC devices may display higher power conversion efficiency than traditional spiro-OMeTAD-based devices and exhibit better thermal stability.

The present invention thus relates to organic hole transporting materials (HTMs) useful to increase power conversion efficiency (PCE) of perovskite solar cells (PSCs). In particular, the present invention relates to spirobifluorene compounds 7 or 8 as given below: 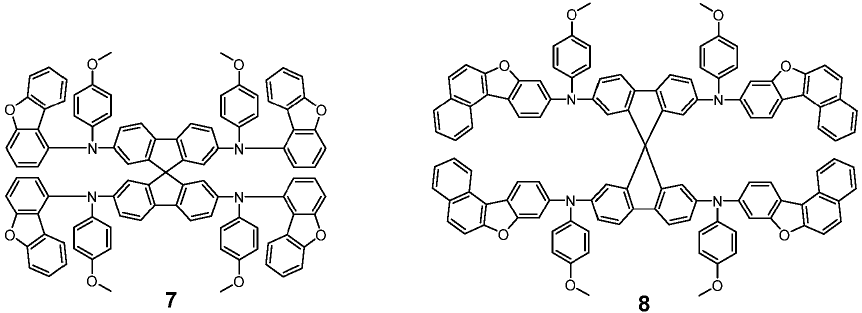 as well as to methods of preparing the above two compounds as defined in the appended claims, the use of the above two compounds 7 and 8 as a hole transport material in a perovskite solar cell, and a perovskite solar cell comprising the following layers:
(a) a transparent conducting layer;
(c) a perovskite layer;
(d) a hole transport layer;
(e) an electrode layer,
wherein the hole transport layer (d) contains one of the above two compounds 7 and 8.

### BRIEF DESCRIPTION OF THE DRAWINGS

Features, advantages, and technical and industrial significance of exemplary embodiments of the invention will be described below with reference to the accompanying drawings, in which like signs denote like elements, and wherein:
Figure 1 (Fig. 1) shows a cyclic voltammetry test of the conventional compound spiro-OMeTAD and reference compounds not part of the present invention;
Figure 2 (Fig. 2) shows experimentally determined electrical conductivity of spiro-OMeTAD and reference compounds not part of the present invention;
Figure 3 (Fig. 3) shows a differential thermal test result of spiro-OMeTAD and reference compounds not part of the present invention;
Figure 4 (Fig. 4) contains Figure 4a (Fig. 4a), which is a cross-sectional scanning electron microscope (SEM) image, and Figure 4b (Fig. 4b), which is a corresponding schematic structural diagram of a perovskite solar cell (PSC) device.
Figure 5 (Fig. 5) contains voltage and current density measurements for spiro-OMeTAD and reference compounds not part of the present invention, enabling power conversion efficiency (PCE) to be determined;
Figure 6 (Fig. 6) shows thermal aging tests in which power conversion efficiency (PCE) variation over time is studied, comparing spiro-OMeTAD and reference compounds not part of the present invention;
Figure 7 (Fig. 7) shows pathways of organic synthesis developed for reference compounds 1 to 6 that are not part of the present invention, and compounds 7 and 8 that are part of the present invention .

### DETAILED DESCRIPTION OF EMBODIMENTS

Reference compounds 1 to 6 that are not part of the present invention, and compounds 7 and 8 that are part of the present invention, are spirobifluorene compounds having the following structure: 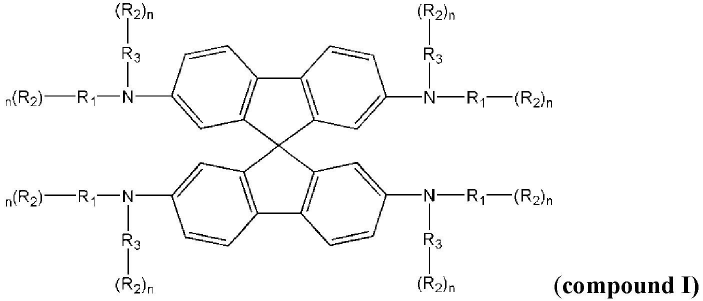 wherein R₁ and R₃ are both ring structures containing one or more aromatic or heteroaromatic rings, R₁ and R₃ not both being phenyl at the same time.

In compounds of the invention, the R₁ and R₃ structures include phenyl.

Compounds of the invention include dibenzofuran (7) joined through its ring position 1 to a N atom of compound I and a (8) napthalene-benzofuran structure:

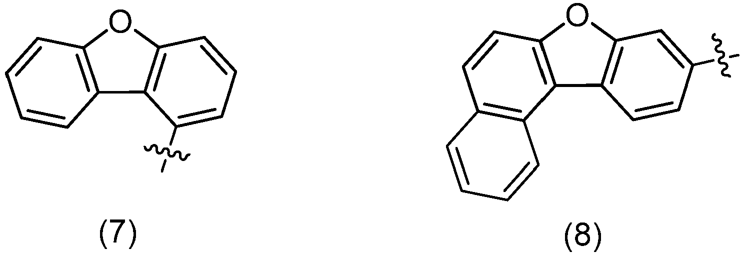

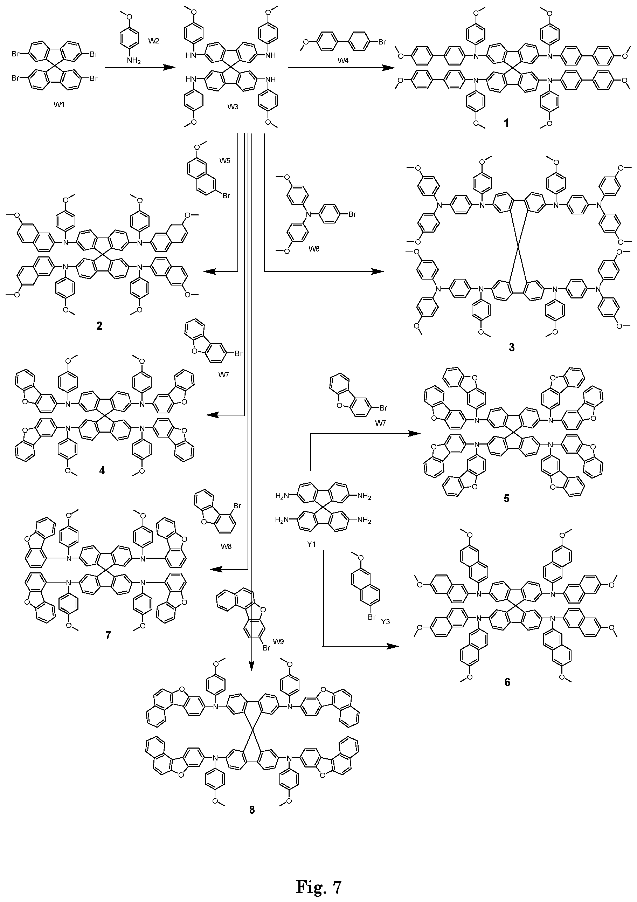

Reference Compounds 1, 2, 3 and 4, not according to the present invention, as well as Compounds 7 and 8 according to the present invention shown in Figure 7, are examples where the groups R₁ and R₃ are different, and Compounds 5 and 6 are examples where the groups R₁ and R₃ are identical. In these specific examples shown in Figure 7 as Compounds 1, 2, 3 and 4 as well as Compounds 7 and 8, one of the -R₁-(R₂)ₙ and -R₃-(R₂)ₙ structures is a p-(MeO-)-phenyl group with a MeO group on the phenyl aromatic group in the para position relative to the nitrogen atom appearing in the general formula of compound (I). In compounds 7 and 8 of the invention, all four of the nitrogen atoms of the spirobifluorene core are joined to one phenyl group (group (1)) and to one other type of group, the phenyl group having a methoxy (MeO-) substituent, in the para-position as in spiro-OMeTAD, which has only such groups attached to the nitrogen atoms of the spirobifluorene core.

In the case where groups R₁ and R₃, as is the case for compounds 7 and 8 of the invention, are different, in the method of synthesis (denoted hereinafter as method I) of the invention, the preparation method comprises the following steps:
(1) 2,2',7,7'-tetrabromo-9,9'-spirobifluorene, a compound H₂N-R₃-(R₂)ₙ, a first palladium-based catalyst, a first alkali metal alcoholate, a first alkylphosphine, and a first aromatic solvent are mixed, and a first Buchwald-Hartwig reaction is carried out to obtain compound II: 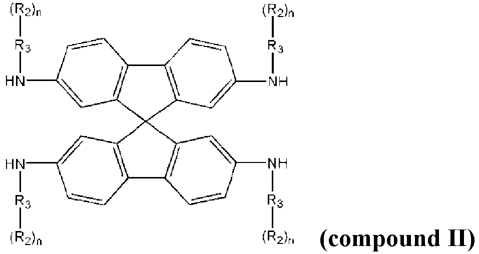
(2) The compound II thus obtained, a compound Br-R₁-(R₂)ₙ, a second palladium-based catalyst, a second alkali metal alcoholate, a second alkyl phosphine and a second aromatic solvent are then mixed to carry out a second Buchwald-Hartwig reaction in order to obtain the sought-after spirobifluorene compound, i.e. one with a terminal aromatic ring extension,
wherein in the method of preparing both of compounds 7 and 8, each (R₂)ₙ-R₃- group in compound II is (p-Me-O)-C₆H₄-, and for preparing compounds 7 and 8, the (R₂)ₙ-R₁-group in the compound Br-R₁-(R₂)ₙ is according to formula (7) or formula (8) below, respectively.

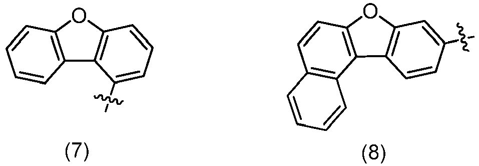

In the present invention, the molar ratio of the 2,2',7,7'-tetrabromo-9,9'-spirobifluorene to NH₂-R₃-(R₂)ₙ in step (1) above is preferably 1:(4~10), more preferably, 1:(6-10), most preferably 1:(8-10).

In the present invention, the first palladium-based catalyst preferably includes one or both of tris(dibenzylideneacetone)dipalladium (Pd₂(dba)₃(0)) and palladium acetate.

In the present invention, the mass ratio of the 2,2',7,7'-tetrabromo-9,9'-spirobifluorene to the first palladium-based catalyst is preferably 1:(0.25~0.4), more preferably 1: (0.3~0.35).

In the present invention, the first metal alcoholate reagent preferably includes an one or both of potassium tert-butoxide and sodium tert-butoxide. In the present invention, the mass ratio of the 2,2',7,7'-tetrabromo-9,9'-spirobifluorene to the first metal alcoholate reagent is preferably 1:(0.8~1.2), more preferably 1: (0.9 to 1.1), more preferably 1:1.

In the present invention, the first alkyl phosphine is preferably tri-tert-butyl phosphine. In the present invention, the molar ratio of the 2,2',7,7'-tetrabromo-9,9'-spirobifluorene to the first alkylphosphine is preferably (20~40):1, more preferably (32~35): 1. In the present invention, the first alkyl phosphine is preferably used in the form of an alkyl phosphine solution, and the solvent in the alkyl phosphine solution is preferably toluene; the concentration of the alkyl phosphine solution is preferably 0.05-0.2 mol/L, more preferably 0.1 to 0.15 mol/L.

In the present invention, the first aromatic solvent preferably includes toluene. The present invention does not specifically limit the amount of the first aromatic solvent, as long as the first Buchwald-Hartwig reaction can be ensured smoothly.

In the present invention, the mixing is not particularly limited, and the raw materials can be mixed uniformly, such as stirring and mixing. In the present invention, degassing (exclusion of air) is preferably performed before the mixing process, and the degassing is preferably performed using a protective atmosphere, the protective atmosphere preferably includes nitrogen or an inert gas, and the inert gas preferably includes helium or argon gas.

In the present invention, the temperature of the first Buchwald-Hartwig reaction is preferably 90 to 120° C, more preferably performed under reflux conditions. The first Buchwald-Hartwig reaction time is preferably 24~48 h, more preferably 30~48 h.

After completing the first Buchwald-Hartwig reaction, the method of the present invention preferably further includes post-treatment (referred to as the first post-treatment), and the first post-treatment preferably includes: diluting the obtained first Buchwald-Hartwig reaction solution with an organic solvent and washing with water, and subsequently the obtained organic phase is dried over anhydrous Na₂SO₄, and then concentrated, the obtained concentrate is purified by column chromatography, and the obtained eluted product is washed with an organic solvent and then dried to obtain a compound II. In the present invention, the organic solvent for dilution at this stage preferably includes dichloromethane and/or ethyl acetate; the volume ratio of the first aromatic solvent to the organic solvent for dilution is preferably 1:(1~3), more preferably is 1: (1.5~2). In the present invention, the number of times of the water washing is preferably 2 to 4 times. The present invention is not particularly limited as regards the procedure for concentration, and a concentration method well-known to those skilled in the art can be used, such as rotary evaporation under reduced pressure. In the present invention, the eluent for column chromatography purification preferably comprises a dichloromethane-petroleum ether mixed solvent, and the volume ratio of dichloromethane and petroleum ether in the dichloromethane-petroleum ether mixed solvent is preferably (6 ~1):1, more preferably (3~4):1. In the present invention, the organic solvent for washing preferably includes an ethyl acetate-petroleum ether mixed solvent, and the volume ratio of ethyl acetate to petroleum ether is preferably 1: (4 ~10), more preferably 1:(5~8). In the present invention, the drying temperature is preferably 50 to 90 °C, more preferably 60 to 80 °C. The present invention does not specifically limit the drying time, and it is sufficient to dry to a constant weight.

After the compound II is obtained, in a method of the present invention, one mixes the compound II, a compound Br-R₁-(R₂)ₙ, the second palladium catalyst, the second alkali metal alcoholate, the second alkyl phosphine and the second aromatic solvent to carry out the second Buchwald-Hartwig reaction to obtain the spirobifluorene compound with terminal extension.

In the present invention, in this second step of synthesis, the molar ratio of the compound II to Br-R₁-(R₂)ₙ is preferably 1:(4~10), more preferably 1:(6~10), further preferably 1:(8~10).

In the present invention, in this second step of synthesis, the second alkyl phosphine is preferably tri-tert-butyl phosphine. In the present invention, the mass ratio of the compound II to the second alkylphosphine is preferably (2-10):1, more preferably (4-8):1.

In the present invention, in this second step of synthesis, the second palladium-based catalyst preferably includes one or both of tris(dibenzylideneacetone)dipalladium (Pd₂(dba)₃(0)) and palladium acetate. In the present invention, the mass ratio of the compound II to the second palladium-based catalyst is preferably (2-10):1, more preferably (3-6):1.

In the present invention, the second alkaline reagent preferably includes an alkali metal alcoholate, more preferably includes one or both of potassium tert-butoxide and sodium tert-butoxide. In the present invention, the mass ratio of the compound II to the second basic reagent is preferably 1:(1~5), more preferably 1:(2~4).

In the present invention, the second aromatic solvent is preferably toluene. The present invention does not specifically limit the amount of the second aromatic solvent, as long as the first Buchwald-Hartwig reaction can be ensured smoothly. The ratio of the amount of the compound II to the volume of the second aromatic solvent is preferably 1 mmol: 60-125 mL.

In the present invention, the conditions of the mixing with the second Buchwald-Hartwig reaction are the same as those of the mixing with the first Buchwald-Hartwig reaction in the preparation process of the aforementioned compound II.

After completing the second Buchwald-Hartwig reaction, the present invention preferably further includes post-treatment (referred to as the second post-treatment), and the second post-treatment preferably includes: diluting the obtained second Buchwald-Hartwig reaction solution and then extracting with an organic solvent, the obtained organic phase is concentrated, and the obtained concentrate is purified by column chromatography to obtain the spirobifluorene compound with end extension. In the present invention, the dilution solvent preferably includes one or more of dichloromethane, chloroform and ethyl acetate, and the dilution ratio is preferably 1 to 2 times. In the present invention, the number of times of the extraction is preferably 4 to 8 times; the organic solvent for extraction preferably includes dichloromethane and/or ethyl acetate; the difference between the first aromatic solvent and the organic solvent for single extraction is the volume ratio of preferably 1:(0.7 to 1.5), more preferably (1 to 1.2):1. The present invention does not have a special limitation on the concentration, and a concentration method well-known to those skilled in the art can be used, such as rotary evaporation under reduced pressure. In the present invention, the eluent for column chromatography purification preferably includes dichloromethane-petroleum ether solvent, and the volume ratio of dichloromethane and petroleum ether in the dichloromethane-petroleum ether solvent is preferably (1~3): (0~3).

Compounds may be prepared, not according to the present invention, wherein the R₁ and R₃ groups are the same, such as Compounds 5 and 6 shown in Figure 7.

For method I, steps 1 and 2, a most preferred mixture of palladium-based catalyst, alkali metal alcoholate and alkylphosphine reagent for the Buchwald-Hartwig reaction is: palladium(II) acetate, potassium tert-butoxide, and tri-tert-butylphosphine. Another preferred mixture is tris(dibenzylideneacetone)dipalladium(0), potassium tert-butoxide, and tri-tert-butylphosphonium tetrafluoroborate.

The present invention provides the application of the spiro-bifluorene compound with an end extension according to the above technical solution as a hole transport material in perovskite solar cells.

### <Perovskite solar cell>

A perovskite solar cell according to the present invention comprises the following layers:
(a) a transparent conducting layer;
(c) a perovskite layer;
(d) a hole transport layer;
(e) an electrode layer

Preferably, the perovskite solar cell can further comprise one or more of: (b) an electron transport layer and (b') an electron-blocking layer. The presence of one or more of these layers leads to a higher device efficiency. The above-mentioned three layers can be disposed in the order of appearance above, with possible extra layers such as an electron transport layer (b) situated between the transparent conducting layer (a) and the perovskite layer (c).

In one embodiment, the perovskite solar cell may have a conventional n-i-p structure comprising a transparent conducting layer, an electron transport later, a perovskite layer, a hole transport layer and an electrode layer in this order.

In another embodiment, the perovskite solar cell may have an inverted p-i-n structure comprising a transparent conducting layer, a hole transport later, a perovskite layer, an electron transport layer and an electrode layer in this order.

In still another embodiment, the perovskite solar cell can further comprise mesoporous scaffold, in the perovskite layer.

Generally, in the so-called p-i-n or n-i-p structures, the electron (n) or hole (p) blocking layers are situated in the bottom and top of the perovskite layer (i), in a sandwich configuration, with the perovskite layer (i) being in the middle. A mesoporous TiO₂ layer is usually an electron transport layer (on the n side), but it can also be a hole transport layer (p side).

### <(a) Transparent conductive layer>

A transparent conductive layer is not particularly limited and can comprise or consist of, for example, a fluorine-doped tin oxide (FTO), indium tin oxide (ITO), doped zinc oxide, carbon nanotube networks or graphene, and preferably an FTO.

### <(b) Electron transport layer (ETL)> and <(b') Hole-blocking layer>

The electron transport layer is not particularly limited and can comprise or consist of, for example, TiO₂, SnO₂, Nb-doped SnO₂, Sb-doped SnO₂, C60 and C60 derivatives, bathocuproine (BCP), a combination of C60/BCP, and a combination of TiO₂/SnO₂ bilayer.

Among these materials, a combination of TiO₂/SnO₂ layer is preferred for n-i-p configuration, and a combination of C60/BCP is preferred for p-i-n configuration.

In the context of a perovskite solar cell, it is possible for there to be (b') a hole-blocking layer. An optional hole blocking layer can be, for example, 2,9-dimethyl-4, 7-diphenylphenanthroline (BCP), TiO₂, or ZnO. The electron transport layer may be the same as the hole-blocking layer. In preferred embodiments of the present invention, where n-type semiconductive materials are used, a same layer (b)/(b') will transport electrons and block holes at the same time.

When the electron transport layer (ETL) material is TiO₂, TiO₂ behaves as both electron transport and hole blocking, given that the layer is compact. However if the ETL material is mesoporous, the perovskite solution infiltrates and touches the FTO electrode (which will produce losses). In order to avoid the direct contact between perovskite and FTO when the ETL material is mesoporous, typically a compact layer of TiO₂ may be advantageously incorporated between FTO and mesoporous-TiO₂ (blocking layer).

### <(c) Perovskite layer>

The perovskite layers most favorably contain "organic-inorganic hybrid perovskites", hereinafter also referred to as "hybrid perovskites", which are ABX₃ materials with A being organic cation groups such as methylammonium (MA), butylammonium (BA), formamidinium (FA), or the alkali metals Cs or Rb, and their mixtures; B being a metal such as Pb, Sn, Bi, Cu, Ag and their mixtures; and X being halides such as Cl, Br, I and their mixtures. More generally, a perovskite can be represented by the formula Al_{w}A2ₓA3_{(1-w-x)}Bl_{y}B2_{(1-y)}X1_{z}X2_{(3-z)}, wherein A1, A2 and A3 are the same or different organic cation groups such as methylammonium (MA), butylammonium (BA), formamidinium (FA), or the alkali metal cations Cs⁺ or Rb⁺; w and x are both 0 or more and 1 or less; B1 and B2 are the same or different metals such as Pb, Sn, Bi, Cu or Ag; y is 0 or more and 1 or less; X1 and X2 are the same or different halides such as Cl, Br or I; and z is 0 or more and 1 or less. Preferred organic-inorganic hybrid perovskites in the present invention have lead (Pb) as the majority metal B in molar terms, and most preferably lead (Pb) is the sole metal B, so that the perovskite is of the form Al_{w}A2ₓA3_{(1-w-x)}PbX1_{z}X2_{(3-z)}. More preferably X1 and X2 are Br and I, with I being most preferred.

Layered perovskites contain additional bulkier molecules such as phenethylamine (PEA) or phenyethylammonium, hexylammonium (HA) and longer chain ammonium species, which confine different perovskite domains into different repeating units. Specific examples given here comprise inorganic metal-halide multi-layered perovskites. These examples are non-limiting.

In an appropriate perovskite (precursor) material for the present invention, a combination of caesium (Cs) metal cation and organic cations such as formamidinium (FA) and/or methylammonium (MA) may be used. A particularly preferred cation system is the triple cation system Cs-FA-MA. The anions used as perovskite (precursor) material for the present invention may appropriately contain a mixture of iodide (I) and bromide (Br) anions. A particularly preferred peroxide precursor system is (Cs_{0.05}MA_{0.05}FA_{0.9})PbI₃.

### <(c') Passivation layer>

In a preferred option, a passivation layer may be present between the perovskite layer and the hole transport layer (HTL) containing hole transport materials (HTM). A preferred component of the passivation layer is phenethylammonium iodide (C₆H₅)-(CH₂)₂NH₃⁺I⁻ (PEAI). A passivation layer containing a material such as PEAI layer may be used to reduce defects in the perovskite layer, for improving the voltage and fill factor in solar cells, as discussed in literature reference https://doi.org/10.1038/s41566-019-0398-2. In perovskite solar cells (PSCs), another small molecular passivation layer material may be used such as octyl ammonium iodide (OAI), as referred to in https://doi.org/10.1038/s41586-021-03406-5.

### <(d') Electron-blocking layer>

An optional electron blocking layer can be, for example, AlGaN. The electron-blocking layer can be the same as the hole transport layer (d), a layer thus used both to block electrons and transport holes. In perovksite solar cells of the present invention, with compounds similar to Spiro-OMeTAD as hole transport materials for the hole transport layer, with their LUMO being higher than perovskite, they also block electron transport.

### <(d) Hole transport layer>

The hole transport layer in the perovskite solar cell according to the present invention contains a spirobifluorene compound of formula (I), in the form of compound (7) or (8) whose synthesis is described in the present invention.

### <(e) Electrode layer>

The electrode layer is not particularly limited and can comprise or consist of, for example, Au, C, Ag, Cu or Al. Among these, Au is preferred on the laboratory scale since it is very efficient but expensive. For industrial applications, carbon and Cu are preferred.

In a typical solar cell structure of the present invention, the structure (a)-(b/b')-(c)-(c')-(d)-(e) is preferably:

FTO/TiO₂/SnO₂/perovskite/PEAI/Spiro-OMeTAD/Au,

electrode layer (e) here being Au on the laboratory scale, carbon and Cu being preferred for industrial applications.

### EXAMPLES

Figure 7 show the synthetic procedures used to transform a functionalized spirobifluorene starting material, specifically compound W1 (2,2',7,7'-tetrabromo-9,9'-spirobifluorene) and compound Y1 (2,2',7,7'-tetraamino-9,9'-bibispirfluorene), into new compounds of the present invention.

### Compound 1 (not according to the invention):

Synthesis of compound W3: compound W1 (2,2',7,7'-tetrabromo-9,9'-spirobifluorene, 1.6 mmol, 1 g), compound W2 (p-aminoanisole, 16 mmol, 1.96 g), Pd₂(dba)₃(0) (300 mg), potassium tert-butoxide (1 g), tri-tert-butylphosphine (0.1 mol/L solution in toluene, 0.5 mL) and toluene (20 mL) were added into a 50 mL round-bottomed flask, then poured into 30 mL of toluene, degassed with argon, the reaction system was refluxed under stirring for 48 h, cooled to room temperature, diluted with 30 mL of dichloromethane, and then 50 mL of water was added. After extraction 4 times with dichloromethane, the obtained organic phase was dried with anhydrous Na₂SO₄, the solvent was removed by a rotary evaporator, and purified by column chromatography (dichloromethane: petroleum ether volume ratio=3:1), the obtained product was used with the mixture of ethyl acetate and petroleum ether (ethyl acetate: petroleum ether volume ratio = 1:4) was washed twice, and dried to constant weight at 60 °C to obtain compound W3 (white solid, 0.96 g, yield 75 %). Structural characterization data of compound W3: ¹H NMR (400 MHz, DMSO) δ 7.71 (s, 4H), 7.53 (d, *J* = 8.3 Hz, 4H), 6.97 - 6.87 (m, 12H), 6.86 - 6.75 (m, 8H), 6.21 (d, *J* = 2.0 Hz, 4H), 3.66 (s, 12H).

Synthesis of compound 1: under nitrogen protection, compound W3 (0.3 mmol, 0.24 g), compound W4 (2.4 mmol, 0.63 g), palladium(II) acetate (40 mg), potassium tert-butoxide (400 mg), tri-tert-butyl phosphine (30 mg) and toluene (20 mL) were added to a 50 mL round-bottomed flask, degassed with nitrogen, heated to reflux for 48 h with stirring, cooled to room temperature, and 20 mL of dichloromethane was added to the reaction system, and then diluted with 50 mL of water. After extraction 4 times with dichloromethane, the obtained organic phases were combined, concentrated by rotary evaporation, and the obtained crude product purified by column chromatography (dichloromethane: petroleum ether volume ratio = 3:1), to obtain compound 1 (white product, 323 mg, 70% yield). Structural characterization data of compound 1: ¹H NMR (500 MHz, DMSO-d₆) δ 7.57 (d, *J* = 8.2 Hz, 4H), 7.47 (d, *J* = 8.8 Hz, 8H), 7.37 (d, *J* = 8.7 Hz, 8H), 6.95 (d, *J =* 8.8 Hz, 16H), 6.88 - 6.75 (m, 20H), 6.40 (d, *J =* 2.0 Hz, 4H), 3.75 (s, 12H), 3.71 (s, 12H). ¹³C NMR (126 MHz, DMSO) δ 158.88, 156.34, 149.87, 146.98, 146.82, 139.99, 135.63, 133.56, 132.51, 127.57, 127.18, 123.63, 122.12, 121.20, 118.29, 115.28, 114.70, 65.38, 55.53, 55.45.

### Compound 2(not according to the invention):

Synthesis of compound 2: Under nitrogen protection, compound W3 (0.3 mmol, 0.24 g), compound W5 (2.4 mmol, 0.57 g), palladium(II) acetate (40 mg), potassium tert-butoxide (400 mg), tri-tert-butyl phosphine (30 mg) and toluene (20 mL) were added to a 50 mL round-bottomed flask, degassed with nitrogen, heated to reflux for 48 h with stirring, cooled to room temperature, diluted with 20 mL of dichloromethane, and then 50 mL of water was added. After extracting 4 times with dichloromethane, the obtained organic phases were combined and concentrated by rotary evaporation, and the obtained crude product was purified by column chromatography (dichloromethane:petroleum ether volume ratio=2:1) to obtain compound 2 (White product, 156 mg, 36% yield). Compound 2 structural characterization data: ¹H NMR (500 MHz, DMSO-d₆) δ 7.68 (d, *J =* 9.7 Hz, 4H), 7.62 (d, *J* = 8.9 Hz, 4H), 7.55 (d, *J* = 8.3 Hz, 4H), 7.11 (d, *J* = 1.7 Hz, 4H), 7.03 - 6.94 (m, 16H), 6.88 (m, 16H), 6.45 (d, *J* = 1.9 Hz, 4H), 3.75 (s, 12H), 3.71 (s, 12H). ¹³C NMR (125 MHz, CDCl3) δ 158.13, 156.10, 150.01, 146.81, 146.52, 146.45, 140.87, 136.46, 135.72, 129.01, 128.28, 126.81, 124.72, 124.24, 120.36, 120.33, 119.27, 116.61, 114.75, 104.97, 65.72, 55.52, 55.20.

### Compound 3(not according to the invention):

Synthesis of compound 3: Under nitrogen protection, compound W3 (0.16 mmol, 0.13 g), compound W6 (4-bromo-4',4"-dimethoxytriphenylamine, 1 mmol, 0.38 g), palladium(II) acetate (40 mg), potassium tert-butoxide (400 mg), tri-tert-butylphosphine (30 mg), and toluene (20 mL) were added to a 50 mL round bottom flask, degassed with nitrogen, and heated with stirring, refluxed for 48 h, cooled to room temperature, diluted with 20 mL of dichloromethane. Then, 50 mL of water was added, followed by extraction 4 times with dichloromethane. The obtained organic phases were combined and concentrated by rotary evaporation, and the obtained crude product was purified by column chromatography (dichloromethane) to give compound 3 (161 mg, 50% yield). Structural characterization data of compound 3: ¹H NMR (500 MHz, DMSO-) δ 7.55 (d, *J* = 8.2 Hz, 4H), 6.96 - 6.55 (m, 68H), 6.19 (m, 4H), 3.67 (s, 24H), 3.64 (s, 12H). Elemental Analysis: C, 79.30; H, 5.60; N, 5.56; found: C, 79.33; H, 5.59; N, 5.55.

### Compound 4 (not according to the invention):

Synthesis of compound 4: Under nitrogen protection, compound W3 (0.3 mmol, 0.24 g), compound W7 (2.4 mmol, 0.59 g), palladium(II) acetate (50 mg), potassium tert-butoxide (500 mg), tri-tert-butyl phosphine (40 mg) and toluene (20 mL) were added to a 50 mL round-bottomed flask, degassed with nitrogen, heated to reflux for 48 h with stirring, cooled to room temperature, diluted with 20 mL of dichloromethane. Then, 50 mL of water was added, followed by extracting 4 times with dichloromethane. The obtained organic phases were combined and concentrated by rotary evaporation, and the obtained crude product was purified by column chromatography (dichloromethane) to obtain compound 4 (231 mg, yield 53%). ). Structural characterization data of compound 4: ¹H NMR (500 MHz, DMSO) δ 7.97 (d, J = 7.7 Hz, 4H), 7.72 (d, J = 2.2 Hz, 4H), 7.65 (d, J = 8.3 Hz, 4H), 7.555 - 7.39 (m, 12H), 7.29 (t, J = 7.6 Hz, 4H), 7.04 (dd, J = 8.8, 2.2 Hz, 4H), 6.90 (m, 16H), 6.72 (dd, J = 8.3, 2.0 Hz, 4H), 6.35 (d, J = 2.0 Hz, 4H), 3.73 (s, 12H). Elemental Analysis: C, 82.77; H, 4.68; N, 3.82; found: C, 82.74; H, 4.69; N, 3.84.

### Compound 5 (not according to the invention):

Synthesis of compound 5: Under nitrogen protection, compound Y1 (2,2',7,7'-tetraamino-9,9'-bibispirfluorene, 0.17 mmol, 64 mg), compound W7 (2.22 mmol, 0.55 g), palladium(II) acetate (40 mg), potassium tert-butoxide (300 mg), tri-tert-butylphosphine (30 mg) and toluene (20 mL) were added to a 50 mL round bottom flask, degassed with nitrogen, heated to reflux for 48 h under stirring conditions, cooled to room temperature, and diluted with 20 mL of ethyl acetate. Then, 50 mL of water was added, followed by extraction with dichloromethane 4 times. The obtained organic phases were combined and concentrated by rotary evaporation. The product was purified by column chromatography (dichloromethane: petroleum ether volume ratio=2:3) to obtain compound 5 (yellow product, 48 mg, yield 17%). Structural characterization data of compound 5: ¹H NMR (500 MHz, DMSO) δ 7.96 (d, J = 7.6 Hz, 8H), 7.83 (d, J = 1.9 Hz, 8H), 7.66 (d, J = 8.3 Hz, 8H), 7.60 (d, J = 8.8 Hz, 8H), 7.47 (t, J = 7.8 Hz, 8H), 7.38 (d, J = 8.3 Hz, 4H), 7.28 (t, J = 7.5 Hz, 8H), 7.14 (m, 8H), 6.75 (d, J = 8.3 Hz, 4H), 6.55 (d, J = 2.0 Hz, 4H). Elemental Analysis: C, 85.19; H, 4.02; N, 3.28; found: C, 85.16; H, 4.00; N, 3.29.

### Compound 6 (not according to the invention):

Synthesis of compound 6: Under the protection of nitrogen, compound Y1 (2,2',7,7'-tetraamino-9,9'-bibispirfluorene, 0.25 mmol, 94 mg), compound Y3 (3.4 mmol, 0.81 g), palladium(II) acetate (50 mg), potassium tert-butoxide (400 mg), tri-tert-butylphosphine (30 mg) and toluene (20 mL) were added to a 50 mL round bottom flask, degassed with nitrogen, which was heated to reflux for 48 h under stirring conditions, cooled to room temperature, and diluted with 20 mL of ethyl acetate. Then, 50 mL of water was added, followed by extraction with dichloromethane 4 times. The obtained organic phases were combined and concentrated by rotary evaporation. The product was purified by column chromatography (dichloromethane: petroleum ether volume ratio=1:1) to obtain compound 7 (yellow product, 81 mg, yield 20%). Structural characterization data of compound 7: ¹H NMR (500 MHz, DMSO) δ 7.74 (d, *J =* 8.9 Hz, 8H), 7.55 (d, *J =* 9.1 Hz, 8H), 7.48 (d, *J =* 8.3 Hz, 4H), 7.36 (d, *J =* 1.3 Hz, 8H), 7.29 (d, *J =* 2.1 Hz, 8H), 7.16 (m, 8H), 7.07 (m, 8H), 6.84 (dd, *J =* 8.2, 1.8 Hz, 4H), 6.53 (s, 4H), 3.85 (s, 24H). Elemental Analysis: C, 83.47; H, 5.21; N, 3.45; found: C, 83.45; H, 5.20; N, 3.41.

### Compound 7 (according to the invention):

Under nitrogen protection, compound W3 (0.3 mmol, 0.24 g), compound W8 (2.4 mmol, 0.59 g), tri-tert-butylphosphonium tetrafluoroborate (30 mg), potassium tert-butoxide (400 mg), and toluene (20 mL) were added to a 50 mL round-bottomed flask, degassed with nitrogen, Then, tris(dibenzylideneacetone)dipalladium(0) (30 mg) was added to the reaction mixture, which was then heated to reflux for 48 h with stirring, cooled to room temperature, and diluted with 20 mL of dichloromethane. Then, 50 mL of water was added, followed by extracting 4 times with dichloromethane. The obtained organic phases were combined and concentrated by rotary evaporation, and the obtained crude product was purified by column chromatography (dichloromethane:petroleum ether volume ratio=2:1) to obtain compound 7 (277 mg, yield 54%). Structural characterization data of compound 7: ¹H NMR (500 MHz, DMSO) δ 7.51 (m, 12H), 7.34 (d, *J =* 8.3 Hz, 4H), 7.21 (t, *J =* 7.8 Hz, 4H), 7.12 - 6.99 (m, 12H), 6.98 - 6.85 (m, 12H), 6.80 (t, *J =* 7.6 Hz, 4H), 6.64 (d, *J =* 1.9 Hz, 4H), 6.55 (dd, *J =* 8.3, 1.9 Hz, 4H), 3.74 (s, 12H).

### Compound 8 (according to the invention):

Under nitrogen protection, compound W3 (0.2 mmol, 0.16 g), compound W9 (1.6 mmol, 0.48 g), tri-tert-butylphosphonium tetrafluoroborate (25 mg), potassium tert-butoxide (400 mg), and toluene (20 mL) were added to a 50 mL round-bottomed flask, degassed with nitrogen, Then, tris(dibenzylideneacetone)dipalladium(0) (25 mg) was added to the reaction mixture, which was then heated to reflux for 48 h with stirring, cooled to room temperature, and diluted with 20 mL of dichloromethane. Then, 50 mL of water was added, followed by extracting 4 times with dichloromethane. The obtained organic phases were combined and concentrated by rotary evaporation, and the obtained crude product was purified by column chromatography (dichloromethane:petroleum ether volume ratio=2:1) to obtain compound 8 (275 mg, yield 83%). Structural characterization data of compound 8: ¹H NMR (500 MHz, DMSO) δ 8.53 (d, *J =* 8.2 Hz, 4H), 8.30 (d, *J* = 8.6 Hz, 4H), 8.04 (d, *J =* 7.9 Hz, 4H), 7.90 (d, *J =* 9.1 Hz, 4H), 7.68 (m, 8H), 7.61 (d, *J* = 8.1 Hz, 4H), 7.53 (t, *J =* 7.4 Hz, 4H), 7.14 - 7.05 (m, 12H), 6.97 (m, 16H), 6.54 (d, 4H), 3.74 (s, 12H).

### 1. Measuring energy level, conductivity, and glass transition temperature measurement

### 1.1 Energy level measurement

Cyclic voltaic curves of the compounds were performed using a CHI660D electrochemical analyzer (CH Instruments, Inc., China), using a common three-electrode system consisting of a platinum wire counter electrode, a platinum working electrode, and a calomel reference electrode.

The redox potentials of the compounds were tested in dichloromethane solution of tetrabutylammonium hexafluorophosphate (the concentration of tetrabutylammonium hexafluorophosphate was 0.1 mol/L), and the scan rate was 50 mV·s-1.

### 1.2 Conductivity measurement

The devices tested for conductivity included FTO/hole transport layer/metal electrode. The hole transport layer was prepared by spin coating, and the hole transport material solution used in spin coating was prepared by dissolving each compound in dry chlorobenzene (concentration of 60 mg/mL). At the same time, 5.7 µL of bistrifluoromethanesulfonimide lithium salt (520 mg/mL in acetonitrile solution) and 1.4 µL of tris(2-(1H-pyrazol-1-yl) pyridine) cobalt (300 mg/mL in acetonitrile) and 9.4 µL of 4-tert-butylpyridine.

### 1.3 Thermal stability measurement

The differential thermal analysis test was carried out on a differential scanning calorimeter (METATEST E3-300) under nitrogen conditions with a heating rate of 10 °C/min.

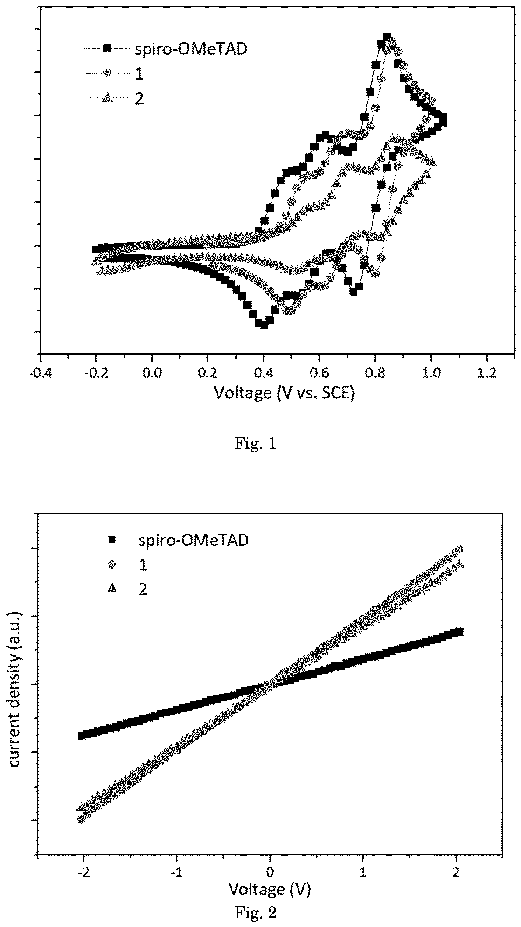

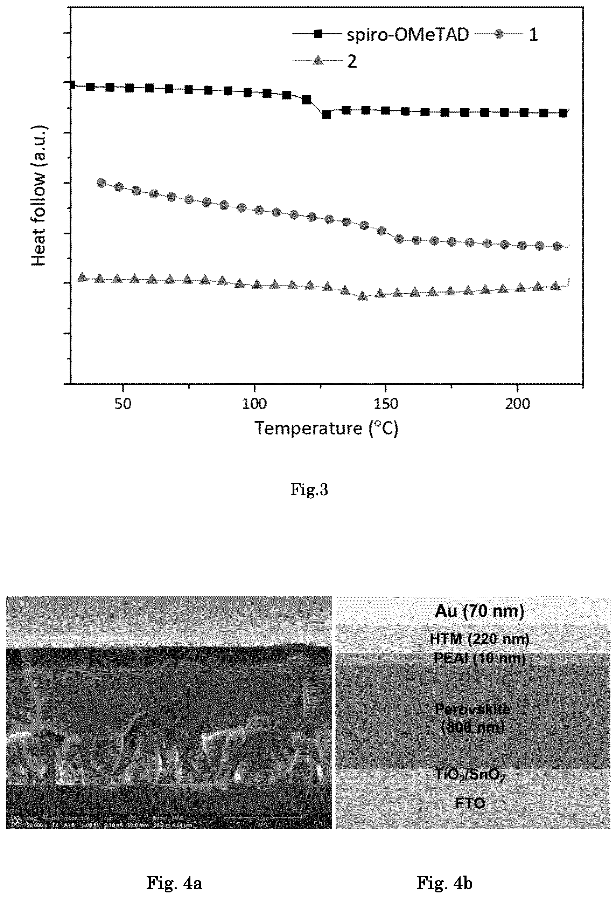

The results of compounds tested are shown in Figs. 1 to 3, and Table 1. Among them, Fig. 1 is the cyclic voltammetry test of spiro-OMeTAD, Compound 1 and Compound 2, and the HOMO energy level of each compound is calculated according to Fig. 1. Fig. 2 show test results for spiro-OMeTAD, Compound 1 and Compound 2, and the electrical conductivity of each compound is calculated according to Figure 2. Figure 3 is a differential thermal test result of spiro-OMeTAD, Compound 1 and Compound 2.

**Table 1: Energy level, conductivity, and glass transition temperature (T_{g}) of compound spiro-OMeTAD, compound 1 and compound 2**

| Samples | HOMO level (eV) | Conductivity (S·cm⁻²) | T_{g} (°C) |
|---|---|---|---|
| spiro-OMeTAD | -5.10 | 4.1×10⁻⁴ | 122 |
| Compound 1 | -5.18 | 9.7×10⁻⁴ | 149 |
| Compound 2 | -5.22 | 8.9×10⁻⁴ | 132 |

As shown in Figures 1 to 3, and Table 1, the HOMO energy level of the spirobifluorene compounds with terminal extensions is significantly lower than that of spiro-OMeTAD. In addition, the electrical conductivity is significantly higher than that of spiro-OMeTAD, and the glass transition temperature is significantly higher. As compared to the spiro-OMeTAD, it can be noted that the spirobifluorene compound with terminal extension has high stability and high electrical conductivity.

### 2. Photovoltaic performance of spiro-OMeTAD, Compound 1 and Compound 2.

A PSC device with an architecture of FTO glass/compact TiO₂ layer (c-TiO₂) /compact SnO₂ layer (c-SnO₂)/Cs_{0.05}MA_{0.05}FA_{0.9}PbI₃ (PVK)/spiro-OMeTAD (HTM)/Au structure was fabricated. The patterned FTO substrate (Asahi FTO glass, 12-13 Ω cm⁻²) was sequentially cleaned with detergent (5% Hellmanex in water), deionized water, acetone, and isopropanol in the ultrasonic bath for 30 min, respectively. The FTO substrate was then further cleaned with ultraviolet-ozone surface treatment for 15 min. The compact TiO₂ layer (c-TiO₂) and SnO₂ layer were sequentially deposited on the clean FTO substrate by the chemical bath deposition (CBD) method. The substrate was annealed on a hotplate at 190 °C for 60 min. The perovskite precursor solution (1.4 M) was prepared by adding 645.4 mg of PbI₂, 216.7 mg of formamidinium iodide (FAI), 11.1 mg of methylamonium iodide (MAI), and 11.8 mg of CsCl into 200 µL of N, N'-dimethylsulfoxide (DMSO) and 800 µL of dimethyformamide (DMF) mixture. After UV-ozone treatment of the substrates for 15 min, the perovskite precursor solution was spin-coated onto the surface of the FTO/c-TiO₂/c-SnO₂ substrate at 1000 rpm for 10 s, accelerated to 5000 rpm for 5s and maintained at this speed for 20 s. This process was carried out in an N₂-filled glove box. Then, the substrate was placed in a homemade rapid vacuum drying equipment. After pumping for 20 s, a brown, transparent perovskite film with a mirror-like surface was obtained. The fresh perovskite layer was annealed at 100 °C for 1 h and then at 150 °C for 10 min. Afterwards, 60 µL of PEAI solution (5 mg/mL in isopropanol) was spin-coated on the perovskite film at 5000 rpm for 30 s. A hole transport layer was deposited on the perovskite film by depositing a doped spiro-OMeTAD solution at 3000 rpm for 30 s. The doped spiro-OMeTAD solution was prepared by dissolving 105 mg of spiro-OMeTAD and 41 µL of 4-tert-butylpyridine in 1343 µL of chlorobenzene with an additional 25 µL of bis(trifluoromethane)sulfonimide lithium salt solution (517 mg/mL in acetonitrile) and 19 µL of cobalt-complex solution (376 mg/mL in acetonitrile). Finally, a ~70 nm-thick gold layer was evaporated on the spiro-OMeTAD layer as the back electrode.
**Fig. 4a** is a cross-sectional SEM image and **Fig. 4b** a corresponding schematic structural diagram of PSC device. The active areas of the PSCs are covered a metal mask with a size of 0.06 cm².

**Table 2: Photovoltaic parameters of PSC devices of spiro-OMeTAD, Compound 1 and Compound 2**

| Devices | Voltage (V) | Current density (mA·cm⁻²) | Fill factor (%) | Efficiency (%) |
|---|---|---|---|---|
| spiro-OMeTAD | 1.140 | 25.95 | 83.0 | 24.56 |
| Compound 1 | 1.143 | 26.11 | 85.0 | 25.37 |
| Compound 2 | 1.160 | 26.45 | 82.4 | 25.27 |

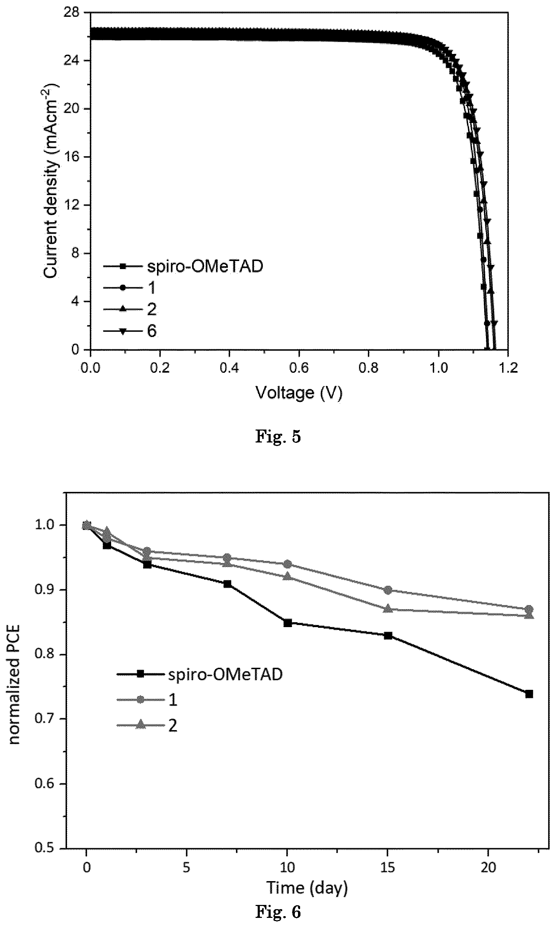

It can be seen from Fig. 5 and Table 2 that the power conversion efficiency of the spirobifluorene compound with terminal extension is higher than that of spiro-OMeTAD.

### 3. The stability measurement of spiro-OMeTAD, Compound 1 and Compound 2.

The PSC devices based on different hole transport materials were stored in an environment with a temperature of 65 °C for thermal aging tests. The test results are shown in Figure 6. It can be seen from Fig. 6 that the device stability of devices based on the spirobifluorene compound with terminal extension is significantly enhanced compared to the spiro-OMeTAD-based devices.

## Claims

1. Spirobifluorene compound which is one of the following compounds 7 or 8:

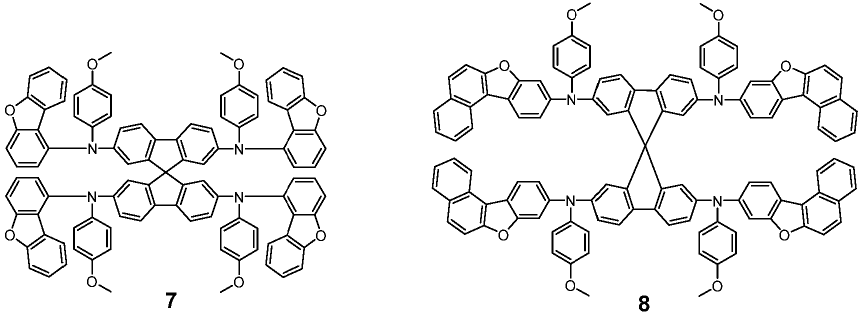

2. Method of preparing a compound according to claim 1 comprising the following steps (1) and then (2):
(1) 2,2',7,7'-tetrabromo-9,9'-spirobifluorene, a compound H₂N-R₃-(R₂)ₙ, a first palladium-based catalyst, a first alkali metal alcoholate, a first alkylphosphine, and a first aromatic solvent are mixed, and a first Buchwald-Hartwig reaction is carried out to obtain a compound II: 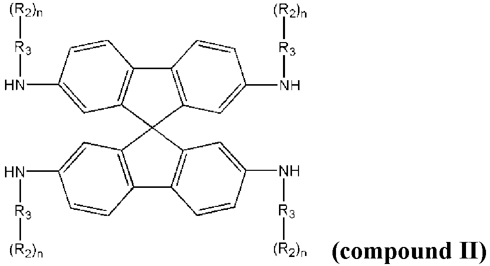
(2) The compound II thus obtained, a compound Br-R₁-(R₂)ₙ, a second palladium-based catalyst, a second alkali metal alcoholate, a second alkyl phosphine and a second aromatic solvent are then mixed to carry out a second Buchwald-Hartwig reaction in order to obtain a spirobifluorene compound according to claim 1,
wherein in the method of preparing both of compounds 7 and 8, each (R₂)ₙ-R₃- group in compound II is (p-Me-O)-C₆H₄-, and for preparing compounds 7 and 8, the (R₂)ₙ-R₁-group in the compound Br-R₁-(R₂)ₙ is according to formula (7) or formula (8) below, respectively.

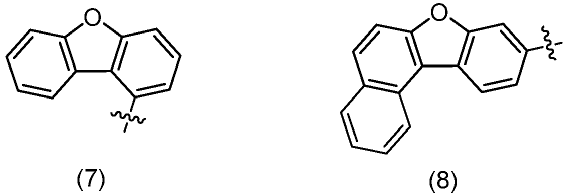

3. Method according to claim 2, in step (1) or (2), wherein the mixture of palladium-based catalyst, alkali metal alcoholate and alkylphosphine for the Buchwald-Hartwig reaction is: (A) palladium(II) acetate, potassium tert-butoxide, and tri-tert-butylphosphine, or (B) tris(dibenzylideneacetone)dipalladium(0), potassium tert-butoxide, and tri-tert-butylphosphonium tetrafluoroborate.

4. Use of a compound (I) according to claim 1 as a hole transport material in a perovskite solar cell.

5. Perovskite solar cell comprising the following layers:
(a) a transparent conducting layer;
(c) a perovskite layer;
(d) a hole transport layer;
(e) an electrode layer,
wherein the hole transport layer (d) contains a compound (I) according to claim 1.

6. Perovskite solar cell according to claim 5, further comprising one or more of: (b) an electron transport layer; and (b') an electron-blocking layer.

## Patentansprüche

1. Spirobifluorenverbindung, die eine der nachstehenden Verbindungen 7 oder 8 ist:

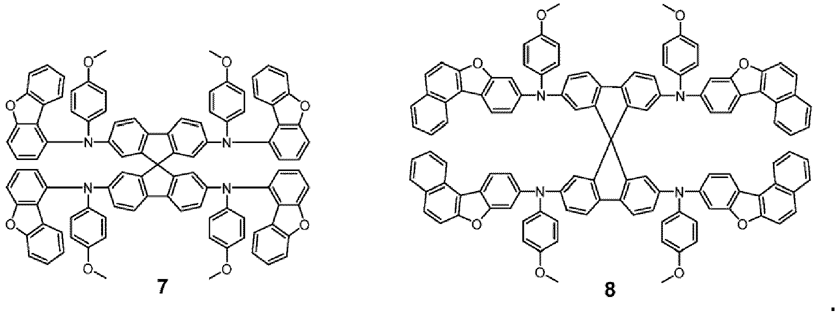

2. Verfahren zum Herstellen einer Verbindung nach Anspruch 1, das die nachstehenden Schritte (1) und dann (2) umfasst:
(1) 2,2',7,7'-Tetrabrom-9,9'-spirobifluoren, eine Verbindung H₂N-R₃-(R₂)ₙ, ein erster Katalysator auf Palladiumbasis, ein erstes Alkalimetallalkoholat, ein erstes Alkylphosphin und ein erstes aromatisches Lösungsmittel werden gemischt und eine erste Buchwald-Hartwig-Reaktion wird durchgeführt, um eine Verbindung II zu erhalten: 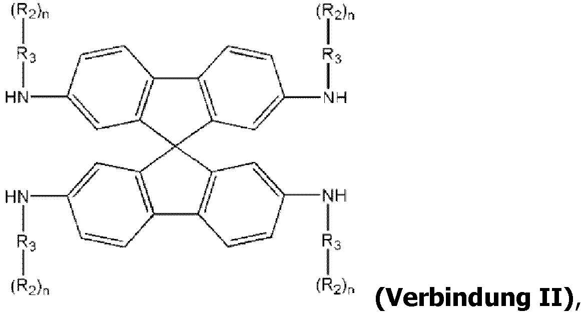
(2) die auf diese Weise erhaltene Verbindung II, eine Verbindung Br-R₁-(R₂)ₙ, ein zweiter Katalysator auf Palladiumbasis, ein zweites Alkalimetallalkoholat, ein zweites Alkylphosphin und ein zweites aromatisches Lösungsmittel werden dann gemischt, um eine zweite Buchwald-Hartwig-Reaktion durchzuführen, um eine Spirobifluorenverbindung nach Anspruch 1 zu erhalten,
wobei bei dem Verfahren zum Herstellen sowohl von Verbindung 7 als auch 8 jede (R₂)ₙ-R₃-Gruppe in Verbindung II (p-Me-O)-C₆H₄- ist und zum Herstellen von Verbindung 7 und 8 die (R₂)ₙ-R₁-Gruppe in der Verbindung Br-R₁-(R₂)ₙ gemäß der nachstehenden Formel (7) bzw. Formel (8) ist.

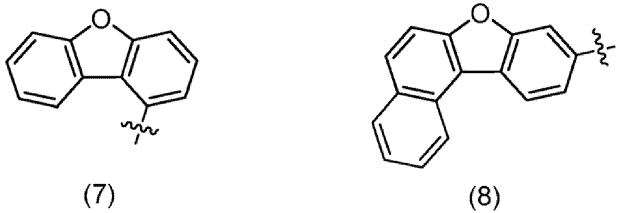

3. Verfahren nach Anspruch 2, wobei in Schritt (1) oder (2) das Gemisch von Katalysator auf Palladiumbasis, Alkalimetallalkoholat und Alkylphosphin für die Buchwald-Hartwig-Reaktion ist: (A) Palladium(II)acetat, Kalium-tert-butoxid und Tri-tert-butylphosphin oder (B) Tris(dibenzylidenaceton)dipalladium(0), Kalium-tert-butoxid und Tri-tert-butylphosphoniumtetrafluorborat.

4. Verwendung einer Verbindung (I) nach Anspruch 1 als Lochtransportmaterial in einer Perowskitsolarzelle.

5. Perowskitsolarzelle, das die folgenden Schichten umfasst:
(a) eine transparente Leitschicht;
(c) eine Perowskitschicht;
(d) eine Lochtransportschicht;
(e) eine Elektrodenschicht,
wobei die Lochtransportschicht (d) eine Verbindung (I) nach Anspruch 1 enthält.

6. Perowskitsolarzelle nach Anspruch 5, die ferner eines oder mehrere umfasst aus: (b) einer Elektronentransportschicht; und (b') einer Elektronensperrschicht.

## Revendications

1. Composé spirobifluorène qui est l'un des composés 7 et 8 suivants :

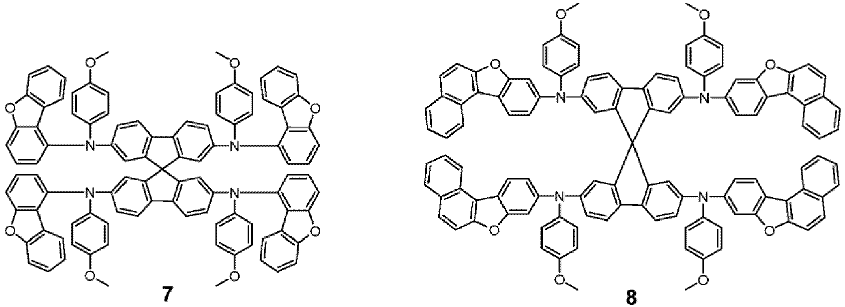

2. Procédé de préparation d'un composé selon la revendication 1, comprenant les étapes (1) puis (2) suivantes :
(1) du 2,2',7,7'-tétrabromo-9,9'-spirobifluorène, un composé H₂N-R₃-(R₂)ₙ, un premier catalyseur à base de palladium, un premier alkylate de métal alcalin, une première alkylphosphine, et un premier solvant aromatique sont mélangés, et une première réaction de Buchwald-Hartwig est mise en œuvre pour que soit obtenu un composé II : 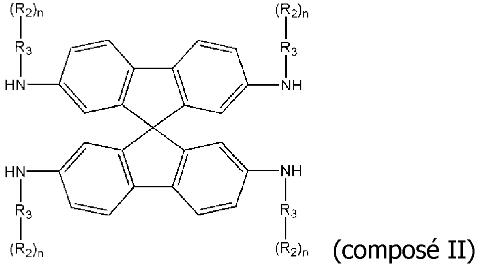
(2) le composé II ainsi obtenu, un composé Br-R₁-(R₂)ₙ, un deuxième catalyseur à base de palladium, un deuxième alkylate de métal alcalin, une deuxième alkylphosphine et un deuxième solvant aromatique sont ensuite mélangés pour que soit mise en œuvre une deuxième réaction de Buchwald-Hartwig afin que soit obtenu un composé spirobifluorène selon la revendication 1,
dans lequel, dans le procédé de préparation des deux composés 7 et 8, chaque groupe (R₂)ₙ-R₃- dans le composé II est (p-Me-O)-C₆H₄-, et pour la préparation des composés 7 et 8, le groupe (R₂)ₙ-R₁- dans le composé Br-R₁-(R₂)ₙ est selon la formule (7) ou la formule (8) ci-dessous respectivement :

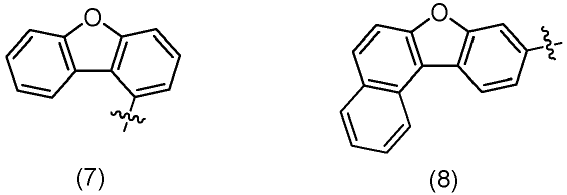

3. Procédé selon la revendication 2, dans l'étape (1) ou (2), dans lequel le mélange de catalyseur à base de palladium, d'alkylate de métal alcalin et d'alkylphosphine pour la réaction de Buchwald-Hartwig est le suivant : (A) acétate de palladium(II), tert-butylate de potassium, et tri-tert-butylphosphine, ou (B) tris(dibenzylidène-acétone)dipalladium(0), tert-butylate de potassium, et tétrafluoroborate de tri-tert-butylphosphonium.

4. Utilisation d'un composé (I) selon la revendication 1 en tant que matériau de transport de trous dans une cellule solaire à perovskite.

5. Cellule solaire à perovskite comprenant les couches suivantes :
(a) une couche conductrice transparente ;
(c) une couche de perovskite ;
(d) une couche de transport de trous ;
(e) une couche d'électrodes,
dans laquelle la couche de transport de trous (d) contient un composé (I) selon la revendication 1.

6. Cellule solaire à perovskite selon la revendication 5, comprenant en outre une ou plusieurs parmi : (b) une couche de transport d'électrons ; et (b') une couche de blocage d'électrons.
